# EUROPÄISCHE PATENTSCHRIFT

(11) **EP 2 347 754 B1**
(45) Veröffentlichungstag und Bekanntmachung des Hinweises auf die Patenterteilung: **24.01.2018**
(21) Anmeldenummer: 10172277.5
(22) Anmeldetag: 09.08.2010
(51) Int. Cl.: A61K 8/41, A61K 8/49, A61Q 11/00

(54) **Mund- und Zahnpflege- und -Reinigungsmittel mit Alkylpyridiniumsalzen I**
Oral and tooth care and cleaning agents with alkylpyridinium salts I
Produit de nettoyage et d'entretien de la bouche et des dents doté d'un de sels d'alkylpyridinium I

(30) Priorität: 16.12.2009 DE 102009054782
(43) Veröffentlichungstag der Anmeldung: 27.07.2011
(73) Patentinhaber: Henkel AG & Co. KGaA, 40589 Düsseldorf (DE)
(72) Erfinder: Adomat, Christel, 40591, Düsseldorf (DE); Adelmann, Barbara, 40597, Düsseldorf (DE); Barth, Adolf Peter, 42781, Haan (DE)

(56) Entgegenhaltungen:
- EP-A1- 0 420 408
- US-A1- 2004 052 754
- DATABASE GNPD [Online] MINTEL; Januar 2008 (2008-01), "Sensitive Mouthwash", XP002725991, Database accession no. 843044

## Beschreibung

Die Erfindung betrifft Zubereitungen zur Mund- und Zahnpflege- und -reinigung, die aufgrund von speziellen Inhaltsstoffen eine ausgezeichnete zahnfleischpflegende und -erhaltende Wirkung besitzen.

Zahnreinigungsmittel sind in verschiedenen Formen auf dem Markt und dienen in erster Linie der Reinigung der Zahnoberfläche und der Vorbeugung von Zahn- und Zahnfleischerkrankungen. Sie enthalten üblicherweise eine Kombination aus Poliermitteln, Feuchthaltemitteln, Tensiden, Bindemitteln, Aromastoffen und fluoridhaltigen sowie antimikrobiellen Wirkstoffen. Neben Zahnpulvern, die wegen ihrer erhöhten Abrasivität eine untergeordnete Rolle spielen, werden Zahnreinigungsmittel vor allem in Pasten-, Creme- und transluzenter oder transparenter Gelform angeboten. In den letzten Jahren haben auch Liquid- oder Flüssigzahncremes und Mundwässer zunehmend an Bedeutung gewonnen.

Neben der Reinigung der Zähne erwartet der Verbraucher von den gattungsgemäßen Produkten auch eine Pflege der Zähne und der Mundhöhle. So sind insbesondere ein "sauberes" Gefühl, d.h. eine glatte und glänzende Zahnoberfläche sowie ein frisches Gefühl im Mund wesentliche Aspekte für den Kaufanreiz von Zubereitungen zur Mund- und Zahnpflege- und -reinigung. Ein erfolgreiches Mittel der gattungsgemäßen Art sollte daher die Zähne gründlich reinigen, ohne den Zahn oder die Zahnoberfläche zu schädigen und gleichzeitig Mundgeruch verringern und/oder verhindern.

Mundgeruch, auch Halitosis oder "Foetor ex ore" genannt, kann verschiedene Ursachen haben. Bei gesunden Menschen wird Mundgeruch jedoch in den meisten Fällen durch Bakterien im Mund- bzw. Rachenraum hervorgerufen, die Körperzellen oder Nahrungsreste verstoffwechseln, wobei als Abbauprodukte von Proteinen u. a. flüchtige Schwefelverbindungen ("volatile sulfur compounds", VSC) entstehen, die für den üblen Geruch verantwortlich gemacht werden.

Diese Schwefelverbindungen sind insbesondere: H₂S = Schwefelwasserstoff (zu etwa 30 %), CH₃-S-H = Methylmercaptan (zu etwa 60 %) und CH₃-S-CH₃ = Dimethylsulfid (zu etwa 10 %). Die flüchtigen Schwefelverbindungen sind teilweise sehr aggressiv und können das Zahnfleisch und die Mundschleimhaut schädigen. So ist beispielsweise bekannt, daß Schwefelwasserstoff und Methylmercaptan die Durchlässigkeit der Mundschleimhaut erhöhen und so Zahnfleischerkrankungen Vorschub leisten können.

Zahnfleischerkrankungen stellen einen hohen Risikofaktor für die Gesundheit dar. Nach Untersuchungen der Weltgesundheitsorganisation (WHO) leiden über 50 % der erwachsenen Bundesbürger an dringend behandlungsbedürftigen Parodontopathien. Unter Parodontitis versteht man eine durch Bakterien hervorgerufene entzündliche Veränderung des den Zahn umgebenden Gewebes, besonders des Kieferknochens. Eine Parodontitis (= mit Beteiligung der Zahnfleischtaschen und des Knochens) entwickelt sich immer aus einer Zahnfleischentzündung (Gingivitis) und befällt zuerst die der Reinigung am schlechtesten zugänglichen Zahnzwischenräume. Die sich in der Zahnfleischtasche befindlichen Bakterien wirken durch ihre Stoffwechselprodukte, insbesondere durch die von Ihnen produzierten VSC, destruktiv auf Zahnfleisch, Zahnsubstanz und Knochen ein und verstärken so ständig die bereits bestehende Parodontitis.

Es hat nicht an Versuchen gefehlt, Mundgeruch und entzündliche Zustände in der Mundhöhle zu bekämpfen, und eine Vielzahl von Agentien wird zu diesem Zweck in Zubereitungen zur Mund- und Zahnpflege und -reinigung eingesetzt. Als antibakterielle Mittel finden beispielsweise Triclosan, Zinnsalze oder Chlorhexidin Verwendung. Der Einsatz dieser Produkte kann jedoch in Einzelfällen mit Nachteilen verbunden sein, die teils geschmacklicher Natur sind, teils ästhetische Gründe haben, da das Produkt und/oder die Zähne durch übermäßigen Gebrauch der Agentien verfärbt werden können. In Einzelfällen kann die Bekämpfung der Bakterien nicht ausreichend sein, um einen Rückgang des entzündlichen Zustandes zu erreichen.

Besonders gut für die orale Anwendung geeignete antibakterielle Mittel sind Cetylpyridiniumchlorid und Chlorhexidin. Alkypyridiniumchloride und Chlorophenylbiguanide besitzen aber den Nachteil, in oralen Anwendungszubereitungen oft nicht stabil einarbeitbar zu sein oder zu Geschmacksbeeinträchtigungen zu führen.

Das europäische Patent EP 0 910 333 B1 schlägt zur Lösung der Stabilitätsprobleme vor, Cetylpyridiniumchlorid (CPC) gemeinsam mit speziellen Betaintensiden, insbesondere mit Cocoamidopropylbetain, einzusetzen.

Die US2004/052754 A1 offenbart Mundspüllösungen, die 0,2 Gew.-% Cetylpyridniumchlorid, 5 Gew.-% Sorbitol, über 50 Gew.-% Wasser und 2 Gew.-% Kaliumsorbat sowie Lauryliminodipropionat-Tocopherylphosphat enthalten.

Es besteht nach wie vor das Bedürfnis, Halitosis zu bekämpfen und Wirkstoffe oder Wirkstoffkombinationen dafür bereitzustellen, die frei von den genannten Nachteilen sind. Zudem besteht der Wunsch, CPC auch ohne den zwingenden Einsatz von Betaintensiden stabil und geschmacksneutral in Zusammensetzungen zur oralen Applikation einzuarbeiten.

Die Aufgabe der vorliegenden Erfindung bestand darin, Zubereitungen zur Mund- und Zahnpflege und -reinigung bereitzustellen, die Mundgeruch wirksam bekämpfen und gegen Gingivitis und Parodontitis wirksam sind. Dabei sollten in einem Aspekt der Erfindung die Nachteile des Einsatzes von Triclosan, Zinnsalzen oder Chlorhexidin vermieden werden können. In einem weiteren Aspekt der Erfindung sollte der stabile und geschmacksneutrale Einsatz von CPC auch ohne den zwingenden Einsatz von Betaintensiden ermöglicht werden.

Es wurde nun gefunden, daß sich bestimmte anionische Tenside zur Lösung des vorstehend genannten Aufgabenkomplexes eignen.

Gegenstand der vorliegenden Erfindung ist in einer ersten Ausführungsform ein Mund- und Zahnpflege- und -reinigungsmittel, enthaltend
a) mindestens ein Alkypyridiniumchlorid und/oder-bromid
b) 0,1 bis 0,8 Gew.-% mindestens eines Tensids der Formel

   R-N((CH₂)₂COO- M1⁺))(CH₂)₂COO- M2⁺
in der R für einen linearen C₈₋₂₄-Alkylrest, M1 für Na und M2 für H stehen.

Mund- und Zahnpflegemittel sowie Mund- und Zahnreinigungsmittel im Sinne der Erfindung sind Mund- und Zahnpulver, Mund- und Zahnpasten, flüssige Mund- und Zahncremes, Mund- und Zahnspülungen sowie Mund- und Zahngele. Eine besonders bevorzugte Ausführungsform der vorliegenden Erfindung sind Mundspüllösungen und Mundwasser, die zum Ausspülen der Mundhöhle verwendet werden.

Die erfindungsgemäßen Mittel enthalten als ersten Inhaltsstoff mindestens ein Alkypyridiniumchlorid und/oder -bromid. Alkylpyridiniumhalogenide sind N-alkylsubstituierte Pyridine mit Halogenidionen als Gegenion. Bevorzugt sind dabei die Chloride.

Als Alkylreste kommen insbesondere C₈-C₂₄-Alkylreste in Frage, wobei lineare C₈-C₂₄-Alkylreste besonders bevorzugt sind. Ganz besonders bevorzugte Reste sind lineare C₁₀-Alkylreste, lineare C₁₂-Alkylreste, lineare C₁₄-Alkylreste, lineare C₁₆-Alkylreste, lineare C₁₈-Alkylreste und Mischungen der entsprechenden Alkylpyridiniumverbindungen, wobei ganz besonders bevorzugte erfindungsgemäße Mund- und Zahnpflege- und -reinigungsmittel - bezogen auf ihr Gewicht - 0,001 - 5 Gew.-%, vorzugsweise 0,005 - 2,5 Gew.-%, besonders bevorzugt 0,01 - 1 Gew.-%, außerordentlich bevorzugt 0,025 - 0,8 Gew.-% und insbesondere 0,05 bis 0,8 Gew.-% Cetylpyridiniumchlorid und/oder Laurylpyridiniumchlorid und/oder 1-Hexadecylpyridiniumchlorid oder deren Mischungen, enthalten. Als zweite wesentliche Komponente enthalten die erfindungsgemäßen Mittel mindestens ein Tensid der Formel R-N((CH₂)₂COO- M1⁺))(CH₂)₂COO⁻ M2⁺, in der R für einen linearen C₈₋₂₄-Alkylrest, M1 für Na und M2 für H stehen

Auch weitere zusätzliche oberflächenaktive Substanzen sind in den erfindungsgemäßen Mitteln einsetzbar. Sie dienen beispielsweise zur Unterstützung der Reinigungswirkung und gewünschtenfalls auch zur Entwicklung von Schaum beim Mundspülen sowie zur Stabilisierung von Dispersionen und werden üblicherweise in einer Menge von 0,1-5 Gew.-% eingesetzt.

Geeignete Tenside sind z. B. lineare Natriumalkylsulfate mit 12-18 C-Atomen in der Alkylgruppe. Weitere geeignete Tenside sind Alkalisalze, bevorzugt Natriumsalze von Alkylpolyglycolethersulfat mit 12-16 C-Atomen in der linearen Alkylgruppe und 2-6 Glycolethergruppen im Molekül, von linearem Alkan(C₁₂- C₁₈)-sulfonat, von Sulfobernsteinsäuremonoalkyl(C₁₂-C₁₈)-estern, von sulfatisierten Fettsäuremonoglyceriden, sulfatisierten Fettsäurealkanolamiden, Sulfoessigsäurealkyl(C₁₂-C₁₆ )-estern, Acylsarcosinen, Acyltauriden und Acylisothionaten mit jeweils 8-18 C-Atomen in der Acylgruppe. Auch zwitterionische, ampholytische und nichtionische Tenside sind geeignet, z. B. Oxethylate von Fettsäuremono- und -diglyceriden, von Fettsäure-Sorbitanestern und Alkyl(oligo)-Glucoside sowie Fettsäureamidoalkylbetaine.

Mit besonderem Vorzug werden werden Alkylpolyglycoside als zusätzliche Tenside eingsetzt, da diese die Formulierung zusätzlich stabilisieren. Alkyl- und Alkenyloligoglykoside stellen bekannte nichtionische Tenside der Formel **R¹O-[G]ₚ** dar, in der R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht.

Die Alkyl- und/oder Alkenyloligoglykoside können sich von Aldosen bzw. Ketosen mit 5 oder 6 Kohlen-stoffatomen, vorzugsweise der Glucose ableiten. Die bevorzugten Alkyl- und/oder Alkenyloligoglykoside sind somit Alkyl- und/oder Alkenyloligo**glucoside**. Die Indexzahl p in der allgemeinen Formel **(I)** gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligo-glykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R¹ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R¹ kann sich ferner auch von primären Alkoholen mit 12 bis 22, vorzugsweise 12 bis 14 Kohlenstoffatomen ableiten. Typische Beispiele sind Laurylalkohol, My-ristylalkohol, Cetylalkohol, Palmoleylalkohol, Stearylalkohol, Isostearylalkohol, Oleylalkohol, Elaidyl-alkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden kön-nen. Bevorzugt sind Alkyloligoglucoside auf Basis von gehärtetem C_{12/14}-Kokosalkohol mit einem DP von 1 bis 3.

Zusätzlich zu dem bzw. den Alkypyridiniumchloriden bzw. -bromiden können die erfindungsgemäßen Mittel auch natürliche antibakterielle Verbindungen enthalten. Deren Wirkung wird durch die erfindungsgemäße Komponente a) ebenfalls synergistisch verstärkt, so daß sie auch in kleinen Dosen hocheffektiv wirken. Besonders bevorzugt sind daher auch erfindungsgemäße Mund- und Zahnpflege- und -reinigungsmittel, die zusätzlich Thymol und/oder Eugenol und/oder Eucalyptol und/oder Extrakte von Myrrhe und/oder Extrakte von Salbei und/oder Extrakte von Nelke, und/oder Extrakte von Tee, und/oder Extrakte von Propolis, und/oder Extrakte von Echinacea enthalten.

Die erfindungsgemäßen Mund- und Zahnpflege- und -reinigungsmittel enthalten weitere Inhaltsstoffe. Bevorzugt ist hierbei der Einsatz von so genannten Feuchthaltemitteln, die bei Zahnpasten das Austrocknen verhindern. Bei so genannten Flüssigzahncremes mit fließfähiger Rheologie dienen diese als Matrix und werden in höheren Mengen eingesetzt. Bei Mundwässern und Mundspülungen dienen diese Alkohole als Konsistenzregler und zusätzliche Süßungsmittel. Bei Mundwasser kann auf diese Feuchthaltemittel aber auch ganz verzichtet werden.

Hier sind erfindungsgemäße Mund- und Zahnpflege- und -reinigungsmittel bevorzugt, die - bezogen auf ihr Gewicht - 0,5 bis 60 Gew.-%, vorzugsweise 0,75 bis 55 Gew.-%, besonders bevorzugt 1 bis 50 Gew.-% und insbesondere 1 bis 40 Gew.-% mindestens eines mehrwertigen Alkohols aus der Gruppe Sorbit und/oder Glycerin und/oder 1,2-Propylenglycol.-% oder deren Mischungen enthalten.

Für bestimmte Anwendungsbereiche kann es vorteilhaft sein, nur einen der drei oben genannten Inhaltsstoffe einzusetzen. In den meisten Fällen ist dabei Sorbit bevorzugt. Allerdings können auf anderen Anwendungsgebieten Mischungen von zwei der drei Stoffe oder aller drei Stoffe bevorzugt sein. Besonders vorteilhaft hat sich hier eine Mischung aus Glycerin, Sorbit und 1,2-Propylenglycol in einem Gewichtsverhältnis von 1 : (0,5-1): (0,1-0,5) erwiesen.

Neben Sorbit bzw. Glycerin bzw. 1,2-Propylenglycol eignen sich als weitere mehrwertige Alkohole solche mit mindestens 2 OH-Gruppen, vorzugsweise Mannit, Xylitol, Polyethylenglycol, Polypropylenglycol und deren Mischungen. Unter diesen Verbindungen sind diejenigen mit 2 bis 12 OH-Gruppen und insbesondere diejenigen mit 2, 3, 4, 5, 6 oder 10 OH-Gruppen bevorzugt.

Polyhydroxyverbindungen mit 2 OH-Gruppen sind beispielsweise Glycol (CH₂(OH)CH₂OH) und andere 1,2-Diole wie H-(CH₂)ₙ-CH(OH)CH₂OH mit n = 2, 3, 4, 5, 6, 7, 8, 9, 10, 11, 12, 13, 14, 15, 16, 17, 18, 19, 20. Auch 1,3-Diole wie H-(CH₂)ₙ-CH(OH) CH₂CH₂OH mit n = 1, 2, 3, 4, 5, 6, 7, 8, 9, 10, 11, 12, 13, 14, 15, 16, 17, 18, 19, 20 sind erfindungsgemäß einsetzbar. Die (n,n+1)- bzw. (n,n+2)-Diole mit nicht endständigen OH-Gruppen können ebenfalls eingesetzt werden. Wichtige Vertreter von Polyhydroxyverbindungen mit 2 OH-Gruppen sind auch die Polyethylen- und Polypropylenglycole. Als bevorzugte weitere mehrwertige Alkohole können z. B. Xylit, Propylenglycole, Polyethylenglycole, insbesondere solche mit mittleren Molekulargewichten von 200-800 eingesetzt werden.

Besonders bevorzugt ist der Einsatz von Sorbit, so daß Mittel, die außer Sorbit keine anderen mehrwertigen Alkohole enthalten, besonders bevorzugt sind.

Mund und Zahnpflege- und -reinigungsmittel können darüber hinaus mit besonderem Vorzug Antikaries-Wirkstoffe enthalten. Diese können beispielsweise aus organischen oder anorganischen Fluoriden ausgewählt sein, z. B. aus Natriumfluorid, Kaliumfluorid, Natriummonofluorphosphat und Natriumfluorosilikat. Auch Zinkfluorid, Zinn-(II)-fluorid sind bevorzugt. Bevorzugt sollte eine Menge von 0,01 - 0,2 Gew.-% Fluor in Form der genannten Verbindungen enthalten sein. Erfindungsgemäße Mund- und Zahnpflege- und -reinigungsmittel, die zusätzlich Antikaries-Wirkstoffe, vorzugsweise Fluorverbindung(en), insbesondere Natriumfluorid, Kaliumfluorid, Natriummonofluorphosphat, Zinkfluorid, Zinnfluorid und Natriumfluorosilikat, vorzugsweise in Mengen von 0,01 bis 5 Gew.%, vorzugsweise in Mengen von 0,01 bis 5 Gew.%, vorzugsweise von 0,02 bis 2,5 Gew.% und insbesondere von 0,04 bis 1,1 Gew.%, jeweils bezogen auf das gesamte Mittel, enthalten, sind erfindungsgemäß bevorzugt.

Die Mund- und Zahnpflegemittel, insbesondere die Zahnpasten, können auch die Unempfindlichkeit der Zähne steigernde Substanzen enthalten, beispielsweise Kaliumsalze wie z. B. Kaliumnitrat, Kaliumcitrat, Kaliumchlorid, Kaliumbicarbonat und Kaliumoxalat. Erfindungsgemäß bevorzugte Mund- und Zahnpflege- und -reinigungsmittel sind dadurch gekennzeichnet, daß sie die Unempfindlichkeit der Zähne steigernde Substanzen, vorzugsweise Kaliumsalze, besonders bevorzugt Kaliumnitrat und/oder Kaliumcitrat und/oder Kaliumchlorid und/oder Kaliumbicarbonat und/oder Kaliumoxalat, vorzugsweise in Mengen von 0,5 bis 20 Gew.%, besonders bevorzugt von 1,0 bis 15 Gew.%, weiter bevorzugt von 1,5 bis 5 Gew.-% und insbesondere von 1,75 bis 2,5 Gew.%, jeweils bezogen auf das gesamte Mittel, enthalten.

Die erfindungsgemäßen Mittel können auch zusätzlich weitere wundheilende und entzündungshemmende Stoffe, z. B. Wirkstoffe gegen Zahnfleischentzündungen, enthalten. Derartige Stoffe können z. B. ausgewählt sein aus Allantoin, Azulen, Kamillenextrakten, Tocopherol, Panthenol, Bisabolol, Salbeiextrakten.

Wenn die erfindungsgemäßen Mittel als Zahnpasten formuliert werden, ist der Einsatz von Putzkörpern (Abrasiva) ebenfalls bevorzugt. Putzkörper sind amorphe, überwiegend anorganische, weitgehend wasserunlösliche, kleinstteilige Pulver, die keine scharfen Kanten aufweisen. Sie begünstigen in Zahn- und Mundpflegemitteln die Reinigung der Zähne und polieren gleichzeitig die Zahnoberfläche (Poliermittel).

Als Poliermittel eignen sich prinzipiell alle für Zahnpasten bekannten Reibkörper, insbesondere solche, die keine Calciumionen enthalten. Bevorzugt geeignete Poliermittel-komponenten sind daher Kieselsäuren, Aluminiumhydroxid, Aluminiumoxid, Natrium-aluminiumsilikate, organische Polymere oder Gemische solcher Reibkörper. Calciumhaltige Polierkomponenten wie z.B. Kreide, Calciumpyrophosphat, Dicalcium-phosphat-dihydrat können aber in Mengen bis zu 5 Gew.-% - bezogen auf die Gesamtzusammensetzung - enthalten sein. Der Gesamtgehalt an Poliermitteln liegt vorzugsweise im Bereich von 5 - 50 Gew.-% des Zahnpflegemittels.

Als weitere Poliermittelkomponente kann auch z.B. Aluminiumoxid in Form von schwach calcinierter Tonerde mit einem Gehalt an - und -Aluminiumoxid in einer Menge von ca. 1 - 5 Gew.-% enthalten sein. Ein solches geeignetes Aluminiumoxid ist unter der Handelsbezeichnung "Poliertonerde P10 feinst" (Giulini Chemie) erhältlich.

Als Poliermittel eignen sich weiter alle für Zahnpasten bekannten Reibkörper wie z. B. Natriumaluminiumsilikate wie z. B. Zeolith A, organische Polymere wie z. B. Polymethacrylat oder Gemische dieser und der vorstehend genannten Reibkörper.

Mund- und Zahnpflege- und reinigungsmittel können z.B. auch Substanzen enthalten, die gegen Plaque und/oder Zahnstein wirksam sind.

Gegen Zahnstein wirksame Stoffe können beispielsweise Chelatbildner sein wie z. B. Ethylendiamintetraessigsäure und deren Natriumsalze, Pyrophosphat- Salze wie die wasserlöslichen Dialkali- oder Tetraalkalimetallpyrophosphat- Salze, z. B. Na₄P₂O₇, K₄P ₂O₇, Na₂K₂P₂O₇, Na₂H₂P₂O₇ und K₂H₂P₂O₇ oder Polyphosphat-Salze, die z. B. aus wasserlöslichen Alkalimethalltripolyphosphaten wie Natriumtripolyphosphat und Kaliumtripolyphosphat ausgewählt sein können.

Erfindungsgemäß bevorzugte Mund- und Zahnpflege- und -reinigungsmittel sind dadurch gekennzeichnet, daß sie zusätzlich Phosphat(e), vorzugsweise Alkalimetallphosphat(e) und insbesondere Natriumtripolyphosphat, vorzugsweise in Mengen von 1 bis 10 Gew.-%, besonders bevorzugt von 2 bis 8 Gew.-% und insbesondere von 3 bis 7 Gew.-%, jeweils bezogen auf das gesamte Mittel, enthalten.

Als Konsistenzregler (bzw. Bindemittel) dienen z. B. natürliche und/oder synthetische wasserlösliche Polymere wie Alginate, Carragheenate, Traganth, Stärke und Stärkeether, Celluloseether wie z. B. Carboxymethylcellulose (Na-Salz), Hydroxyethylcellulose, Methylhydroxypropylcellulose, Guar, Akaziengum, Agar-Agar, Xanthan- Gum, Succinoglycan-Gum, Johannisbrotmehl, Pectine, wasserlösliche Carboxyvinylpolymere (z. B. Carbopol®-Typen), Polyvinylalkohol, Polyvinylpyrrolidon, Polyethylenglycole, insbesondere solche mit Molekulargewichten von 1 500-1 000 000.

Weitere Stoffe, die sich zur Viskositätskontrolle eignen, sind z. B. Schichtsilikate wie z. B. Montmorillonit-Tone, kolloidale Verdickungskieselsäuren wie z. B. Aerogel-Kieselsäuren, pyrogene Kieselsäuren oder feinstvermahlene Fällungskieselsäuren. Es können auch viskositätsstabilisierende Zusätze aus der Gruppe der kationischen, zwitterionischen oder ampholytischen stickstoffhaltigen Tenside, der hydroxypropylsubstituierten Hydrocolloide oder der Polyethylenglycol/Polypropylenglycol- Copolymere mit einem mittleren Molgewicht von 1000 bis 5000 oder eine Kombination der genannten Verbindungen in den Zahnpasten verwendet werden. Die erfindungsgemäßen Mittel, insbesondere die Zahnpasten, können auch Substanzen zur Erhöhung des mineralisierenden Potentials enthalten, beispielsweise calciumhaltige Substanzen wie z. B. Calciumchlorid, Calciumacetat und Dicalciumphosphat-Dihydrat. Die Konzentration der calciumhaltigen Substanz hängt von der Löslichkeit der Substanz und dem Zusammenwirken mit anderen in dem Mund- und Zahnpflegemittel enthaltenen Substanzen ab.

Neben den genannten obligatorischen Komponenten können die erfindungsgemäßen Zahnpflegemittel weitere, an sich bekannte Hilfs- und Zusatzstoffe enthalten. Dabei ist ein Zusatzstoff, der als Zahnpastenkomponente seit langem bekannt ist, in den erfindungsgemäßen Zahnpflegemitteln besonders wirksam: Calcium-glycerophosphat, das Calcium-Salz der Glycerin-1-phosphorsäure oder der Glycerin-2-phosphorsäure oder der zur Glycerin-1-phosphorsäure enantiomeren Glycerin-3-phosphorsäure - oder eines Gemisches dieser Säuren. Die Verbindung hat in Zahnpflegemitteln eine remineralisierende Wirkung, da sie sowohl Calcium- als auch Phosphationen liefert. In den erfindungsgemäßen Zahnpflegemitteln wird Calciumglycerophosphat bevorzugt in Mengen von 0,01 - 1 Gew.-% eingesetzt. Insgesamt können die erfindungsgemäßen Zahnreinigungsmittel übliche Hilfsmittel und Zusatzstoffe in Mengen bis zu 10 Gew.-% enthalten. Die erfindungsgemäßen Zahnpflegemittel können z.B. durch Zusatz von Aromaölen und Süßungsmitteln in ihren organoleptischen Eigenschaften verbessert werden.

Als Aromaöle können alle die für Mund- und Zahnpflegemittel üblichen natürlichen und synthetischen Aromen eingesetzt werden. Natürliche Aromen können sowohl in Form der aus Drogen isolierten natürlichen ätherischen Öle als auch der daraus isolierten Einzelkomponenten enthalten sein.

Geeignete Aromen sind z.B. Pfefferminzöl, Krauseminzöl, Eukalyptusöl, Anisöl, Fenchelöl, Kümmelöl, Menthylacetat, Zimtaldehyd, Anethol, Vanillin, Thymol sowie Mischungen dieser Komponenten.

Geeignete Süßungsmittel sind z.B. Saccharin-Natrium, Natrium-Cyclamat, Sucrose, Lactose, Meltose, Fructose.

Eine bevorzugte Formulierungsvariante für erfindungsgemäße Produkte sind Mundwässer und Mundspüllösungen. Diese enthalten üblicherweise keine Poliermittel oder andere Feststoffe und werden überwiegend wäßrig basiert formuliert.

Erfindungsgemäß bevorzugte Mund- und Zahnpflege- und -reinigungsmittel enthalten - bezogen auf ihr Gewicht - 50 bis 99 Gew.-%, vorzugsweise 60 bis 98,5 Gew.% und insbesondere 75 bis 96 Gew.-% Wasser.

Zusätzlich oder anstelle eines Teils des Wassers können erfindungsgemäße Mundwässer und Mundspüllösungen auch niedere Alkohole, insbesondere Ethanol enthalten. Die bevorzugte Menge an Ethanol beträgt üblicherweise 1 bis 20 Gew.-%, vorzugsweise 2,5 bis 15 Gew.-% und insbesondere 5 bis 10 Gew.-%, wobei die Mengen variieren könne, je nachdem, ob es sich um direkt anzuwendende Produkte oder um Konzentrate handelt, die vor der Anwendung verdünnt werden.

Weitere übliche Hilfs- und Zusatzstoffe für Zahnpasten und Mundwässer oder Mundspüllösungen sind
- Oberflächenaktive Stoffe, bevorzugt anionische, zwitterionische, amphotere, nichtionische Tenside oder eine Kombination mehrerer verschiedener Tenside
- Lösungsmittel und Lösungsvermittler, z.B. niedere einwertige oder mehrwertige Alkohole oder Ether, z.B. Ethanol, 1,2-Propylenglycol, Diethylenglycol oder Butyldi- glycol
- Pigmente, wie z.B. Titandioxid
- Farbstoffe
- Puffersubstanzen, z.B. primäre, sekundäre oder tertiäre Alkaliphosphate oder Citronensäure-/Na-Citrat
- weitere wundheilende oder entzündungshemmende Stoffe, z.B. Allantoin, Harnstoff, Azulen, Kamillewirkstoffe, Acetylsalicylsäurederivate oder Rhodanid
- weitere Vitamine wie z.B. Ascorbinsäure, Biotin, Tocopherol oder Rutin
- Mineralsalze wie z.B. Mangan-, Zink- oder Magnesiumsalze.

Es hat sich gezeigt, daß die erfindungsgemäßen Mund- und Zahnpflege- und -reinigungsmittel in ihrer Leistung weiter gesteigert werden können, wenn die Mittel salivationsfördernde Substanzen enthalten. Insbesondere die antibakterielle Wirkung und mit ihr die Antikarieswirkung und die Wirkung gegen Gingivitis und/oder Parodontitis werden hierdurch verstärkt.

Unter Salivation versteht man die Speichelproduktion und -freisetzung, im weiteren Sinne auch in unphysiologisch erhöhter Menge. Substanzen, die den Speichelfluß anregen und die Speichelmenge und/oder -freisetzung erhöhen, können aus den unterschiedlichsten Stoffklassen stammen.

Eine erfindungsgemäß beispielsweise geeignete Substanz ist das Pilocarpin, das in den erfindungsgemäßen Mund- und Zahnpflege- und -reinigungsmitteln enthalten sein kann.

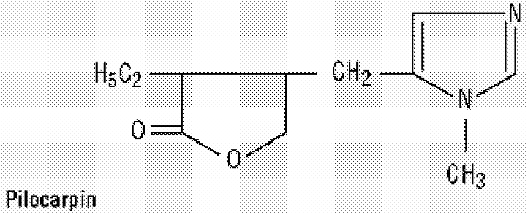

Weitere salivationsfördernde Substanzen sind insbesondere so genannte Scharfstoffe, d.h. scharf schmeckende und/oder ein Gefühl von Wärme erzeugende Substanzen. Erfindungsgemäß bevorzugte Mund- und Zahnpflege- und -reinigungsmittel sind dadurch gekennzeichnet, daß sie als salivationsfördernde Substanz mindestens eine scharf schmeckende und/oder ein Gefühl von Wärme erzeugende Substanz enthalten.

Als salivationsfördernden Inhaltsstoff enthalten die erfindungsgemäßen Erzeugnisse dieser Ausführungsform eine scharf schmeckende und/oder ein Gefühl von Wärme erzeugende Substanz. Diese Substanzen vermitteln dem Anwender einen scharfen, kribbelnden, mundwässernden oder wärmeerzeugenden Effekt, d.h. sie rufen sensorisch einen Wärmeeindruck oder ein Brennen, oder ein Prickeln, Perlen, Kitzeln oder Sprudeln hervor und fördern dadurch den Speichelfluß.

Erfindungsgemäß bevorzugte Erzeugnisse dieser Ausführungsform enthalten die scharf schmeckende(n) und/oder ein Gefühl von Wärme erzeugende(n) Substanz(en) in Mengen von 0,00001 bis 5 Gew.-%, vorzugsweise von 0,0005 bis 2,5 Gew.-%, weiter bevorzugt von 0,001 bis 1 Gew.-%, besonders bevorzugt von 0,005 bis 0,75 Gew.-% und insbesondere von 0,01 bis 0,5 Gew.-%, jeweils bezogen auf das Gewichts des gesamten Mittels.

Als scharf schmeckende oder ein Gefühl von Wärme erzeugende Substanz kann eine Reihe von Stoffen eingesetzt werden. Bevorzugt sind insbesondere N-Alkyl-substituierte Amide von ungesättigten Carbonsäuren, beispielsweise
- 2E,4E-Decadiensäure-N-Methylamid
- 2E,4E-Decadiensäure-N-Ethylamid
- 2E,4E-Decadiensäure-N-n-Propylamid
- 2E,4E-Decadiensäure-N-Isopropylamid
- 2E,4E-Decadiensäure-N-n-Butylamid
- 2E,4E-Decadiensäure-N-(1-Methylpropyl)-amid
- 2E,4E-Decadiensäure-N-Isobutylamid
- 2E,4E-Decadiensäure-N-tert-Butylamid
- 2E,4Z-Decadiensäure-N-Methylamid
- 2E,4Z-Decadiensäure-N-Ethylamid
- 2E,4Z-Decadiensäure-N-n-Propylamid
- 2E,4Z-Decadiensäure-N-Isopropylamid
- 2E,4Z-Decadiensäure-N-n-Butylamid
- 2E,4Z-Decadiensäure-N-(1-Methylpropyl)-amid
- 2E,4Z-Decadiensäure-N-Isobutylamid
- 2E,4Z-Decadiensäure-N-tert-Butylamid
- 2E,4E,8Z-Decatriensäure-N-Methylamid
- 2E,4E,8Z-Decatriensäure-N-Ethylamid
- 2E,4E,8Z-Decatriensäure-N-n-Propylamid
- 2E,4E,8Z-Decatriensäure-N-Isopropylamid
- 2E,4E,8Z-Decatriensäure-N-n-Butylamid
- 2E,4E,8Z-Decatriensäure-N-(1-Methylpropyl)-amid
- 2E,4E,8Z-Decatriensäure-N-Isobutylamid
- 2E,4E,8Z-Decatriensäure-N-tert-Butylamid
- 2E,4Z,8Z-Decatriensäure-N-Methylamid
- 2E,4Z,8Z-Decatriensäure-N-Ethylamid
- 2E,4Z,8Z-Decatriensäure-N-n-Propylamid
- 2E,4Z,8Z-Decatriensäure-N-Isopropylamid
- 2E,4Z,8Z-Decatriensäure-N-n-Butylamid
- 2E,4Z,8Z-Decatriensäure-N-(1-Methylpropyl)-amid
- 2E,4Z,8Z-Decatriensäure-N-Isobutylamid
- 2E,4Z,8Z-Decatriensäure-N-tert-Butylamid
- 2E,4E,8E-Decatriensäure-N-Methylamid
- 2E,4E,8E-Decatriensäure-N-Ethylamid
- 2E,4E,8E-Decatriensäure-N-n-Propylamid
- 2E,4E,8E-Decatriensäure-N-Isopropylamid
- 2E,4E,8E-Decatriensäure-N-n-Butylamid
- 2E,4E,8E-Decatriensäure-N-(1-Methylpropyl)-amid
- 2E,4E,8E-Decatriensäure-N-Isobutylamid
- 2E,4E,8E-Decatriensäure-N-tert-Butylamid
- 2E,4Z,8E-Decatriensäure-N-Methylamid
- 2E,4Z,8E-Decatriensäure-N-Ethylamid
- 2E,4Z,8E-Decatriensäure-N-n-Propylamid
- 2E,4Z,8E-Decatriensäure-N-Isopropylamid
- 2E,4Z,8E-Decatriensäure-N-n-Butylamid
- 2E,4Z,8E-Decatriensäure-N-(1-Methylpropyl)-amid
- 2E,4Z,8E-Decatriensäure-N-Isobutylamid
- 2E,4Z,8E-Decatriensäure-N-tert-Butylamid
- 2E,6Z,8E-Decatriensäure-N-Methylamid
- 2E,6Z,8E-Decatriensäure-N-Ethylamid
- 2E,6Z,8E-Decatriensäure-N-n-Propylamid
- 2E,6Z,8E-Decatriensäure-N-Isopropylamid
- 2E,6Z,8E-Decatriensäure-N-n-Butylamid
- 2E,6Z,8E-Decatriensäure-N-(1-Methylpropyl)-amid
- 2E,6Z,8E-Decatriensäure-N-Isobutylamid
- 2E,6Z,8E-Decatriensäure-N-tert-Butylamid
- 2E,6E,8E-Decatriensäure-N-Methylamid
- 2E,6E,8E-Decatriensäure-N-Ethylamid
- 2E,6E,8E-Decatriensäure-N-n-Propylamid
- 2E,6E,8E-Decatriensäure-N-Isopropylamid
- 2E,6E,8E-Decatriensäure-N-n-Butylamid
- 2E,6E,8E-Decatriensäure-N-(1-Methylpropyl)-amid
- 2E,6E,8E-Decatriensäure-N-Isobutylamid
- 2E,6E,8E-Decatriensäure-N-tert-Butylamid
- 2E,6Z,8Z-Decatriensäure-N-Methylamid
- 2E,6Z,8Z-Decatriensäure-N-Ethylamid
- 2E,6Z,8Z-Decatriensäure-N-n-Propylamid
- 2E,6Z,8Z-Decatriensäure-N-Isopropylamid
- 2E,6Z,8Z-Decatriensäure-N-n-Butylamid
- 2E,6Z,8Z-Decatriensäure-N-(1-Methylpropyl)-amid
- 2E,6Z,8Z-Decatriensäure-N-Isobutylamid
- 2E,6Z,8Z-Decatriensäure-N-tert-Butylamid
- 2E,6E,8Z-Decatriensäure-N-Methylamid
- 2E,6E,8Z-Decatriensäure-N-Ethylamid
- 2E,6E,8Z-Decatriensäure-N-n-Propylamid
- 2E,6E,8Z-Decatriensäure-N-Isopropylamid
- 2E,6E,8Z-Decatriensäure-N-n-Butylamid
- 2E,6E,8Z-Decatriensäure-N-(1-Methylpropyl)-amid
- 2E,6E,8Z-Decatriensäure-N-Isobutylamid
- 2E,6E,8Z-Decatriensäure-N-tert-Butylamid
- 2E,7Z,9E-Undecatriensäure -N-Methylamid
- 2E,7Z,9E-Undecatriensäure -N-Ethylamid
- 2E,7Z,9E-Undecatriensäure -N-n-Propylamid
- 2E,7Z,9E-Undecatriensäure -N-Isopropylamid
- 2E,7Z,9E-Undecatriensäure -N-n-Butylamid
- 2E,7Z,9E-Undecatriensäure -N-(1-Methylpropyl)-amid
- 2E,7Z,9E-Undecatriensäure-N-isobutylamid
- 2E,7Z,9E-Undecatriensäure -N-tert-Butylamid
- 2E,7E,9E-Undecatriensäure -N-Methylamid
- 2E,7E,9E-Undecatriensäure -N-Ethylamid
- 2E,7E,9E-Undecatriensäure -N-n-Propylamid
- 2E,7E,9E-Undecatriensäure -N-Isopropylamid
- 2E,7E,9E-Undecatriensäure -N-n-Butylamid
- 2E,7E,9E-Undecatriensäure -N-(1-Methylpropyl)-amid
- 2E,7E,9E-Undecatriensäure-N-isobutylamid
- 2E,7E,9E-Undecatriensäure -N-tert-Butylamid
- 2E,7Z,9Z-Undecatriensäure -N-Methylamid
- 2E,7Z,9Z-Undecatriensäure -N-Ethylamid
- 2E,7Z,9Z-Undecatriensäure -N-n-Propylamid
- 2E,7Z,9Z-Undecatriensäure -N-Isopropylamid
- 2E,7Z,9Z-Undecatriensäure -N-n-Butylamid
- 2E,7Z,9Z-Undecatriensäure -N-(1-Methylpropyl)-amid
- 2E,7Z,9Z-Undecatriensäure-N-isobutylamid
- 2E,7Z,9Z-Undecatriensäure -N-tert-Butylamid
- 2E,7Z,9E-Undecatriensäure -N-Methylamid
- 2E,7Z,9E-Undecatriensäure -N-Ethylamid
- 2E,7Z,9E-Undecatriensäure -N-n-Propylamid
- 2E,7Z,9E-Undecatriensäure -N-Isopropylamid
- 2E,7Z,9E-Undecatriensäure -N-n-Butylamid
- 2E,7Z,9E-Undecatriensäure -N-(1-Methylpropyl)-amid
- 2E,7Z,9E-Undecatriensäure-N-isobutylamid
- 2E,7Z,9E-Undecatriensäure -N-tert-Butylamid Selbstverständlich sind auch andere Substitutionsmuster am Stickstoffatom möglich und bevorzugt, beispielsweise längerkettige n-Alkylreste (...-N-n-Pentylamid, ...-N-n-Pentylamid, ...-N-n-Pentylamid, ...-N-n-Pentylamid, ...-N-n-Pentylamid, ...-N-n-Hexylamid, ...-N-n-Heptylamid, ...-N-n-Octylamid, ...-N-n-Nonylamid, ...-N-n-Decylamid, ...-N-n-Undecylamid, ...-N-n-Dodecylamid, ...-N-n-Tridecylamid, usw.) oder disubstituierte ...-N,N-Dialkylamide wie ...-N,N-dimethylamid, ...-N,N-diethylamid, ...-N,N-di-n-propylamid, ...-N,N-diisopropylamid, ...-N,N-di-n-butylamid, ...-N,N-di(1-Methylpropyl)amid, ...-N,N-düsobutylamid, ...-N,N-di-tert-butylamid, ...-N,N-methyl-ethylamid, ...-N,N-methyl-n-propylamid, ...-N,N-methyl-isopropylamid, ...-N,N-ethyl-n-propylamid, ...-N,N-ethyl-isopropylamid, usw..

Unter den genannten Verbindungen sind einige im Rahmen der vorliegenden Erfindung besonders bevorzugt. Diese sind nachfolgend aufgeführt:
2E,6Z,8E-Decatriensäure-N-isobutylamid (N-Isobutyl-2E,6Z,8E-decatrienamid, auch Spilanthol oder Affinin genannt): 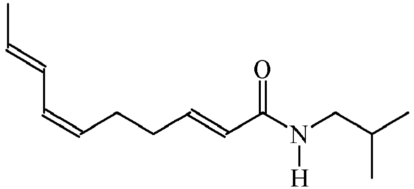
2E,4E,8Z-Decatriensäure-N-isobutylamid (N-Isobutyl-2E,4E,8Z-decatrienamid, auch Isoaffinin genannt): 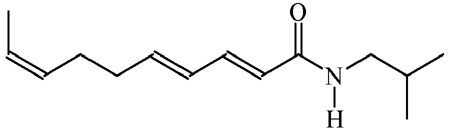
2E,7Z,9E-Undecatriensäure-N-isobutylamid (N-Isobutyl-2E,7Z,9E-undecatrienamid): 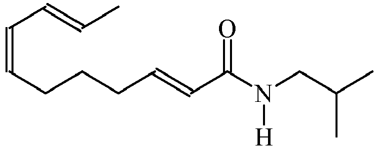
2E,4Z-Decadiensäure-N-isobutylamid (cis-Pellitorin): 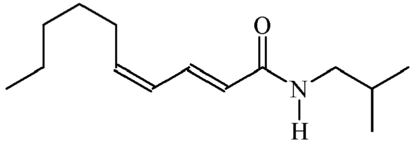
2E,4E-Decadiensäure-N-isobutylamid (trans-Pellitorin): 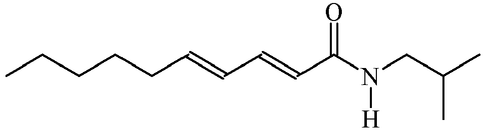
Ferulasäureamide, beispielsweise
   Ferulasäure-N-Vanillylamid: 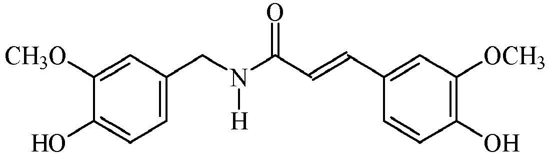
*N*-[2-(4-Hydroxy-3-methoxyphenyl)ethyl]-3-(4-hydroxy-3-methoxy-phenyl)-(2*E*)-propensäureamid (trans-Feruloylmethoxytyramin): 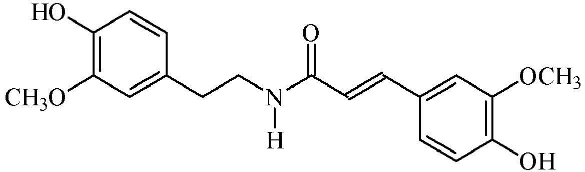
*N*-[2-(4-Hydroxy-3-methoxyphenyl)ethyl]-3-(4-hydroxy-3-methoxy-phenyl)-(2*Z*)-propensäureamid (cis-Feruloylmethoxytyramin): 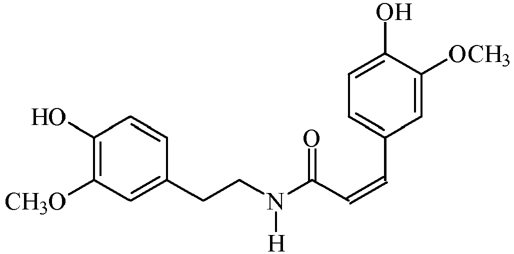
*N*-[2-(4-Hydroxy-3-methoxyphenyl)ethyl]-3-(4-hydroxy-3-methoxy-phenyl)-propansäureamid (Dihydroferuloylmethoxytyramin): 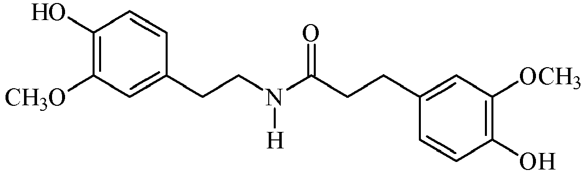
*N*-[2-(3,4-Dihydroxyphenyl)ethyl]-3-(4-hydroxy-3-methoxy-phenyl)-(2*E*)- propensäureamid (trans-Feruloyldopamin): 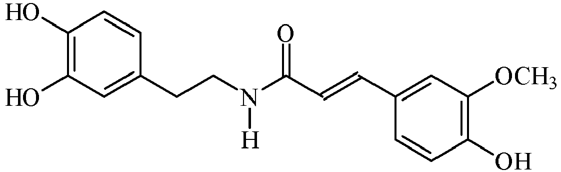
*N*-[2-(3,4-Dihydroxyphenyl) ethyl]-3-(4-hydroxy-3-methoxy-phenyl)-(2Z)-propensäureamid (cis-Feruloyldopamin): 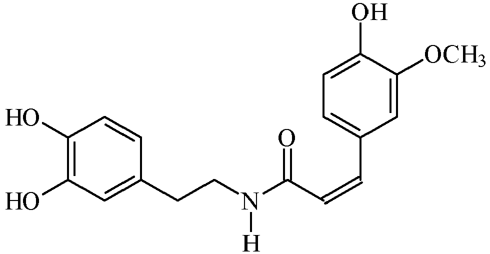
*N*-[2-(4-Hydroxyphenyl)ethyl]-3-(3,4-dihydroxyphenyl)-(2*E*)-propensäureamid (trans-Caffeoyltyramin): 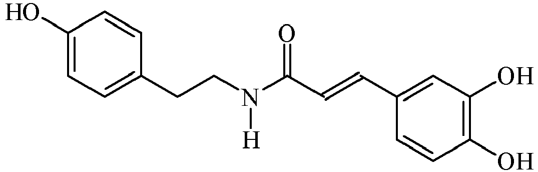
*N*-[2-(4-Hydroxyphenyl)ethyl]-3-(3,4-dihydroxyphenyl)-(2*Z*)-propensäureamid(cis-Caffeoyltyramin): 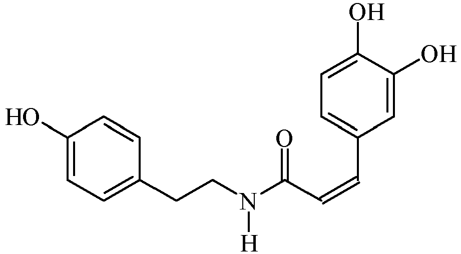
*N*-[2-(3,4-Dimethoxyphenyl)ethyl]-3-(3,4-dimethoxyphenyl)-(2*E*)-propensäureamid (trans-Rubenamin): 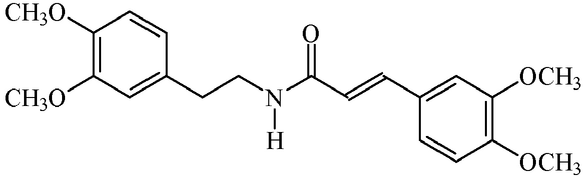
*N*-[2-(3,4-dimethoxyphenyl)ethyl]-3-(3,4-dimethoxy-phenyl)-(2*Z*)-propensäureamid(cis-Rubenamin):

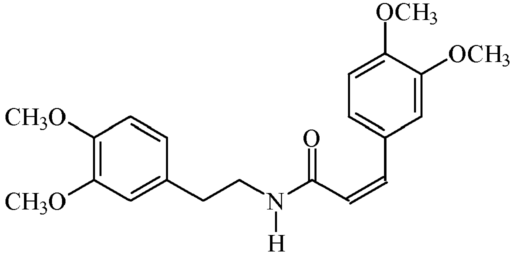

Weitere im Rahmen der vorliegenden Erfindung mit besonderem Vorzug einsetzbare Scharfstoffe sind beispielsweise Extrakte aus Naturpflanzen. Scharf schmeckende pflanzliche Extrakte können alle physiologisch unbedenklichen pflanzlichen Extrakte sein, die einen scharfen oder warmen sensorischen Eindruck hervorrufen. Bevorzugt als scharf schmeckende pflanzliche Extrakte sind beispielsweise Pfefferextrakt (*Piper ssp.,* insbesondere *Piper nigrum*)*,* Wasserpfefferextrakt (*Polygonum ssp.* ,insbesondere *Polygonum hydropiper*)*,* Extrakte aus *Allium* ssp.(insbesondere Zwiebel und Knoblauchextrakte), Extrakte aus Rettich (*Raphanus ssp.*), Meerrettichextrakte (*Cochlearia armoracia*)*,* Extrakte aus schwarzem (*Brassica nigra*), wildem oder gelbem Senf (*Sinapis ssp.,* insbesondere *Sinapis arvensis* und *Sinapis alba*), Bertramwurzel-Extrakte (*Anacyclus ssp.,* insbesondere *Anacyclus pyrethrum*L.), Sonnenhutextrakte (*Echinaceae ssp.),* Extrakten aus Szechuan-Pfeffer (*Zanthoxylum ssp.,* insbesondere *Zanthoxylum piperitum),* Spilanthesextrakt (*Spilanthes ssp.,* insbesondere *Spilanthes acmella*), Chiliextrakt (*Capsicum ssp.,* insbesondere *Capsicum frutescens*), Paradieskörner-Extrakt (*Aframomum ssp*.,insbesondere *Aframomum melegueta*[Rose] K. Schum.),Ingwerextrakt (*Zingiber ssp*.,insbesondere *Zingiber officinale*) und Galangaextrakt (*Kaempferia galanga oderAlpinia galanga*).

Eine besonders geeignete Substanz ist das aus dem Ingwerextrakt stammende Gingerol:

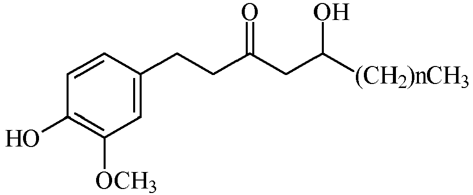

Einsetzbar ist auch N-Ethyl-p-menthan-3-carboxamid (N-Ethyl-5-Methyl-2-isopropylcyclohexancarboxamid):

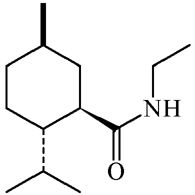

Andere scharf schmeckende oder ein Gefühl von Wärme erzeugende Substanzen können z.B. sein Capsaicin, Dihydrocapsaicin, Gingerol, Paradol, Shogaol, Piperin, Carbonsäure-N-vanillylamide, insbesondere Nonansäure-N- vanillylamid, 2-Alkensäureamide, insbesondere 2-Nonensäure-N-isobutylamid, 2- Nonensäure-N-4-hydroxy-3- methoxyphenylamid, Alkylether von 4-Hydroxy-3- methoxybenzylalkohol, insbesonders 4-Hydroxy-3-methoxybenzyl-n-butylether, Alkylether von 3- Hydroxy-4-methoxybenzylalkohol, Alkylether von 3,4- Dimethoxybenzylalkohol, Alkylether von 3-Ethoxy-4-hydroxybenzylalkohol, Alkylether von 3,4-Methylendioxybenzylalkohol, Nicotinaldehyd, Methylnicotinat, Propylnicotinat, 2-Butoxyethylnicotinat, Benzylnicotinat, 1-Acetoxychavicol, Polygodial oder Isodrimeninol.

Bevorzugte erfindungsgemäße remineralisierende Erzeugnisse sind dadurch gekennzeichnet, daß sie mindestens einen Scharfstoff aus der Gruppe der N-Alkyl-substituierte Amide von ungesättigten Carbonsäuren, vorzugsweise
a. 2E,6Z,8E-Decatriensäure-N-isobutylamid (Spilanthol) und/oder
b. 2E,4E,8Z-Decatriensäure-N-isobutylamid und/oder
c. 2E,7Z,9E-Undecatriensäure-N-isobutylamid und/oder
d. 2E,4Z-Decadiensäure-N-isobutylamid (cis-Pellitorin) und/oder
e. 2E,4E-Decadiensäure-N-isobutylamid (trans-Pellitorin) und/oder
f. Ferulasäure-N-Vanillylamid und/oder
g. *N*-[2-(4-Hydroxy-3-methoxyphenyl)ethyl]-3-(4-hydroxy-3-methoxy-phenyl)-(2*E*)-propensäureamid (trans-Feruloylmethoxytyramin) und/oder
h. *N*-[2-(4-Hydroxy-3-methoxyphenyl)ethyl]-3-(4-hydroxy-3-methoxy-phenyl)-(2*Z*)-propensäureamid (cis-Feruloylmethoxytyramin) und/oder
i. *N*-[2-(4- Hydroxy-3-methoxyphenyl)ethyl]-3-(4-hydroxy-3-methoxy-phenyl)-propansäureamid (Dihydroferuloylmethoxytyramin) und/oder
j. *N*-[2-(3,4- Dihydroxyphenyl)ethyl]-3-(4-hydroxy-3-methoxy-phenyl)-(2*E*)-propensäureamid (trans-Feruloyldopamin) und/oder
k. *N*-[2-(3,4-Dihydroxyphenyl)ethyl]-3-(4-hydroxy-3-methoxy-phenyl)-(2*Z*)-propensäureamid (cis- Feruloyldopamin) und/oder
l. *N*-[2-(4-Hydroxyphenyl)ethyl]-3-(3,4-dihydroxyphenyl)-(2*E*)-propensäureamid (trans-Caffeoyltyramin) und/oder
m. *N*-[2-(4-Hydroxyphenyl)ethyl]-3-(3,4-dihydroxyphenyl)-(2*Z*)- propensäureamid (cis-Caffeoyltyramin) und/oder
n. *N*-[2-(3,4-Dimethoxyphenyl) ethyl]-3-(3,4-dimethoxyphenyl)-(2*E*)-propensäureamid (trans- Rubenamin) und/oder
o. *N*-[2-(3,4-dimethoxyphenyl)ethyl]-3-(3,4- dimethoxy-phenyl)-(2*Z*)-propensäureamid (cis-Rubenamin)
enthalten.

Zusätzlich zu den genannten Scharfstoffen oder an ihrer Stelle können auch weitere scharf schmeckende und/oder ein Gefühl von Wärme erzeugende Substanzen in die erfindungsgemäßen Erzeugnisse eingearbeitet werden.

Als besonders geeignet haben sich im Rahmen der vorliegenden Erfindung alkylsubstituierte Dioxane der Formel 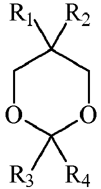 erwiesen, in der R1 und R2 unabhängig voneinander ausgewählt sind aus -H, -CH₃, -CH₂CH₃ und R3 und R4 unabhängig voneinander ausgewählt sind aus -H, -CH₃, -CH₂CH₃, -CH₂CH₂CH₃, - CH(CH₃)₂.

Als weiterhin besonders geeignet haben sich im Rahmen der vorliegenden Erfindung Phenylester der Formel 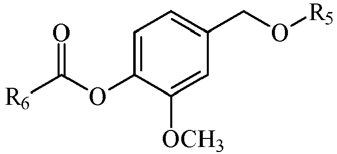 erwiesen, in der R5 für -CH₃ oder einen geradkettigen oder verzweigten Alkyl- oder Alkenylrest mit 2 bis 8 Kohlenstoffatomen und R6 für -CH₃ oder einen geradkettigen oder verzweigten Alkyl- oder Alkenylrest mit 2 bis 8 Kohlenstoffatomen oder eine Alkoxygruppe mit 1 bis 3 Kohlenstoffatomen steht

Als weiterhin besonders geeignet haben sich im Rahmen der vorliegenden Erfindung Carvonacetale der Formel 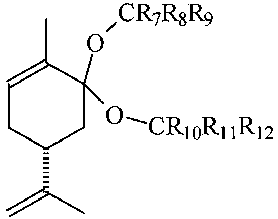 erwiesen, in der R7 bis R12 unabhängig voneinander ausgewählt sind aus -H, -CH₃, - CH₂CH₃, -CH₂CH₂CH₃, -CH(CH₃)₂, -CH₂ CH₂CH₂CH₃, -CH₂CH(CH₃)₂, -CH(CH₃)CH₂CH₃,-C(CH₃)₃ oder R9 und R10 zusammen eine chemische Bindung oder eine Gruppe -(CR13R14)ₓ bedeuten, worin x für die Werte 1 oder 2 steht und R13 und R14 unabhängig voneinander ausgewählt sind aus -H, -CH₃, - CH₂CH₃, -CH₂CH₂CH₃, -CH(CH₃)₂, -CH₂ CH₂CH₂CH₃, -CH₂CH(CH₃)₂, -CH(CH₃)CH₂CH₃,-C(CH₃)₃
Die erfindungsgemäßen Zusammensetzungen lassen sich vorzugsweise als Mundspüllösungen oder Mundwässer formulieren. Ein weiterer Gegenstand der vorliegenden Erfindung ist eine erfindungsgemäße Zubereitung zur Verwendung bei der Vorbeugung vor und Behandlung von Karies und/oder Bekämpfung von Halitosis und/oder zur Behandlung von Gingivitis oder Parodontitis, wobei die Zubereitung in Form einer Mundspüllösung vorliegt, wobei die Mundspüllösung in die Mundhöhle eingebracht und dort für einen Zeitraum von mindestens 10 Sekunden, vorzugsweise mindestens 20 Sekunden und insbesondere mindestens 45 Sekunden belassen wird.

Bezüglich bevorzugter Ausführungsformen der erfindungsgemäß hergerichteten Zubereitung gilt mutatis mutandis das zu den erfindungsgemäßen Mitteln Gesagte.

### Beispiele:

| | |
|---|---|
| Mundspüllösung (alle Angaben in Gew.-%): | |
| Sorbitol 70% | 3,0 |
| Cetylpyridiniumchlorid | 0,05 |
| Natriumfluorid | 0,045 |
| Saccharin Natrium | 0,03 |
| Lauraminodipropionat, Mononatriumsalz | 0,3 |
| Alkylpolyglucosid | 0,05 |
| Aroma | 0,1 |
| Wasser, vollentsalzt | ad 100 |

## Patentansprüche

1. Mund- und Zahnpflege- und -reinigungsmittel, enthaltend - bezogen auf sein Gewicht - in einem oral akzeptablen Träger
a) mindestens ein Alkypyridiniumchlorid und/oder -bromid
b) 0,1 bis 0,8 Gew.-% mindestens eines Tensids der Formel
R-N((CH₂)₂COO⁻ M1⁺))(CH₂)₂COO⁻ M2⁺
in der R für einen linearen C₈₋₂₄-Alkylrest, M1 für Na und M2 für H stehen.

2. Mund- und Zahnpflege- und -reinigungsmittel nach Anspruch 1, **dadurch gekennzeichnet, dass** es - bezogen auf sein Gewicht - - 0,001 - 5 Gew.-%, vorzugsweise 0,005 - 2,5 Gew.-%, besonders bevorzugt 0,01 - 1 Gew.-%, außerordentlich bevorzugt 0,025 - 0,8 Gew.-% und insbesondere 0,05 bis 0,8 Gew.-% Cetylpyridiniumchlorid und/oder Laurylpyridiniumchlorid oder deren Mischungen, enthält.

3. Mund- und Zahnpflege- und -reinigungsmittel nach einem der Ansprüche 1 oder 2, **dadurch gekennzeichnet, dass** es - bezogen auf sein Gewicht - 0,5 bis 60 Gew.-%, vorzugsweise 0,75 bis 55 Gew.-%, besonders bevorzugt 1 bis 50 Gew.-% und insbesondere 2 bis 40 Gew.-% mindestens eines mehrwertigen Alkohols aus der Gruppe Sorbit und/oder Glycerin und/oder 1,2-Propylenglycol.-% oder deren Mischungen enthält.

4. Mund- und Zahnpflege- und -reinigungsmittel nach einem der Ansprüche 1 bis 3, **dadurch gekennzeichnet, dass** es 50 bis 99 Gew.-%, vorzugsweise 60 bis 98,5 Gew-% und insbesondere 75 bis 96 Gew.-% Wasser enthält.

5. Mund und Zahnpflege- und -reinigungsmittel nach einem der Ansprüche 1 bis 4, **dadurch gekennzeichnet, dass** es zusätzlich Antikaries-Wirkstoffe, vorzugsweise Fluorverbindung(en), insbesondere Natriumfluorid, Kaliumfluorid, Natriummonofluorphosphat, Zinkfluorid, Zinnfluorid und Natriumfluorosilikat, vorzugsweise in Mengen von 0,01 bis 5 Gew.%, vorzugsweise von 0,02 bis 2,5 Gew.% und insbesondere von 0,04 bis 1,1 Gew.%, jeweils bezogen auf das gesamte Mittel, enthält.

6. Mund- und Zahnpflege- und -reinigungsmittel nach einem der Ansprüche 1 bis 5, **dadurch gekennzeichnet, dass** es die Unempfindlichkeit der Zähne steigernde Substanzen, vorzugsweise Kaliumsalze, besonders bevorzugt Kaliumnitrat und/oder Kaliumcitrat und/oder Kaliumchlorid und/oder Kaliumbicarbonat und/oder Kaliumoxalat, vorzugsweise in Mengen von 0,5 bis 20 Gew.%, besonders bevorzugt von 1,0 bis 15 Gew.%, weiter bevorzugt von 1,5 bis 5 Gew.-% und insbesondere von 1,75 bis 2,5 Gew.%, jeweils bezogen auf das gesamte Mittel, enthält.

7. Mund- und Zahnpflege- und -reinigungsmittel nach einem der Ansprüche 1 bis 6, **dadurch gekennzeichnet, dass** es zusätzlich Thymol und/oder Eugenol und/oder Eucalyptol und/oder Extrakte von Myrrhe und/oder Extrakte von Salbei und/oder Extrakte von Nelke, und/oder Extrakte von Tee, und/oder Extrakte von Propolis, und/oder Extrakte von Echinacea enthält.

8. Zubereitung nach einem der Ansprüche 1 bis 7 zur Verwendung bei der Vorbeugung vor und Behandlung von Karies und/oder Bekämpfung von Halitosis und/oder zur Behandlung von Gingivitis oder Parodontitis, wobei die Zubereitung in Form einer Mundspüllösung vorliegt, wobei die Mundspüllösung in die Mundhöhle eingebracht und dort für einen Zeitraum von mindestens 10 Sekunden, vorzugsweise mindestens 20 Sekunden und insbesondere mindestens 45 Sekunden belassen wird.

## Claims

1. An oral and dental care and cleaning agent, containing, in an orally acceptable carrier and based on the weight of said agent,
a) at least one alkylpyridinium chloride and/or bromide
b) from 0.1 to 0.8 wt.% of at least one surfactant of formula
R-N((CH₂)₂COO⁻ M1⁺))(CH₂)₂COO⁻ M2⁺
in which R denotes a linear C₈₋₂₄ alkyl functional group, M1 denotes Na and M2 denotes H.

2. The oral and dental care and cleaning agent according to claim 1, **characterized in that** it contains, based on its weight, 0.001-5 wt.%, preferably 0.005-2.5 wt.%, particularly preferably 0.01-1 wt.%, extremely preferably 0.025-0.8 wt.% and in particular 0.05-0.8 wt.%, cetylpyridinium chloride and/or laurylpyridinium chloride or mixtures thereof.

3. The oral and dental care and cleaning agent according to either claim 1 or claim 2, **characterized in that** it contains, based on its weight, from 0.5 to 60 wt.%, preferably from 0.75 to 55 wt.%, particularly preferably from 1 to 50 wt.% and in particular from 2 to 40 wt.%, at least one polyvalent alcohol from the group consisting of sorbitol and/or glycerol and/or 1,2-propylene glycol-% or mixtures thereof.

4. The oral and dental care and cleaning agent according to one of claims 1 to 3, **characterized in that** it contains from 50 to 99 wt.%, preferably from 60 to 98.5 wt.% and in particular from 75 to 96 wt.%, water.

5. The oral and dental care and cleaning agent according to one of claims 1 to 4, **characterized in that** it additionally contains anticaries active ingredients, preferably fluorine compound(s), in particular sodium fluoride, potassium fluoride, sodium monofluorophosphate, zinc fluoride, tin fluoride and sodium fluorosilicate, preferably in amounts of from 0.01 to 5 wt.%, preferably from 0.02 to 2.5 wt.% and in particular from 0.04 to 1.1 wt.%, based in each case on the entire agent.

6. The oral and dental care and cleaning agent according to one of claims 1 to 5, **characterized in that** it contains substances that decrease the sensitivity of teeth, preferably potassium salts, particularly preferably potassium nitrate and/or potassium citrate and/or potassium chloride and/or potassium bicarbonate and/or potassium oxalate, preferably in amounts of from 0.5 to 20 wt.%, particularly preferably from 1.0 to 15 wt.%, more preferably from 1.5 to 5 wt.% and in particular from 1.75 to 2.5 wt.%, based in each case on the entire agent.

7. The oral and dental care and cleaning agent according to one of claims 1 to 6, **characterized in that** it additionally contains thymol and/or eugenol and/or eucalyptol and/or extracts of myrrh and/or extracts of sage and/or extracts of clove and/or extracts of tea, and/or extracts of propolis, and/or extracts of Echinacea.

8. A preparation according to one of claims 1 to 7 for use in preventing and treating caries and/or combating halitosis and/or treating gingivitis or periodontitis, wherein the preparation is in the form of a mouthwash solution, wherein the mouthwash solution is introduced into the oral cavity and is left there for a period of at least 10 seconds, preferably at least 20 seconds and in particular at least 45 seconds.

## Revendications

1. Agent de soin et de nettoyage buccodentaires contenant, sur la base de son poids, dans un support acceptable par voie orale
a) au moins un chlorure et/ou bromure d'alkylpyridinium
b) de 0,1 à 0,8% en poids d'au moins un tensioactif de la formule
R-N((CH₂)₂COO⁻M1⁺))(CH₂)₂COO⁻M2⁺
dans laquelle R représente un radical alkyle en C₈ à C₂₄ linéaire, M1 représente Na et M2 représente H.

2. Agent de soin et de nettoyage buccodentaires selon la revendication 1, **caractérisé en ce qu'**il contient, sur la base de son poids, de 0001 à 5% en poids, de préférence de 0,005 à 2,5% en poids, de manière particulièrement préférée de 0,01 à 1% en poids, de manière très préférée de 0,025 à 0,8% en poids et notamment de 0,05 à 0,8% en poids, de chlorure de cétylpyridinium et/ou de chlorure de laurylpyridinium ou de leurs mélanges.

3. Agent de soin et de nettoyage buccodentaires selon l'une des revendications 1 ou 2, **caractérisé en ce qu'**il contient, sur la base de son poids, de 0,5 à 60% en poids, de préférence de 0,75 à 55% en poids, de manière particulièrement préférée de 1 à 50% en poids et notamment de 2 à 40% en poids, d'au moins un alcool polyvalent du groupe comportant le sorbitol et/ou le glycérol et/ou le 1,2-propylène-glycol ou leurs mélanges.

4. Agent de soin et de nettoyage buccodentaires selon l'une des revendications 1 à 3, **caractérisé en ce qu'**il contient de 50 à 99% en poids, de préférence de 60 à 98,5% en poids et notamment de 75 à 96% en poids, d'eau.

5. Agent de soin et de nettoyage buccodentaires selon l'une des revendications 1 à 4, **caractérisé en ce qu'**il contient en outre des substances anti-carie, de préférence un ou plusieurs composés fluorés, notamment le fluorure de sodium, le fluorure de potassium, le monofluorophosphate de sodium, le fluorure de zinc, le fluorure d'étain et le fluorosilicate de sodium, de préférence dans des quantités de 0,01 à 5% en poids, de préférence de 0,02 à 2,5% et notamment de 0,04 à 1,1% en poids, à chaque fois sur la base de tout l'agent.

6. Agent de soin et de nettoyage buccodentaires selon l'une des revendications 1 à 5, **caractérisé en ce qu'**il contient des substances augmentant l'insensibilité des dents, de préférence des sels de potassium, de façon particulièrement préférée du nitrate de potassium et/ou du citrate de potassium et/ou du chlorure de potassium et/ou du bicarbonate de potassium et/ou de l'oxalate de potassium, de préférence dans des quantités de 0,5 à 20% en poids, de manière particulièrement préférée de 1,0 à 15% en poids, plus préférablement de 1,5 à 5% en poids et notamment de 1,75 à 2,5% en poids, à chaque sur la base de tout l'agent.

7. Agent de soin et de nettoyage buccodentaires selon l'une des revendications 1 à 6, **caractérisé en ce qu'**il contient en outre du thymol et/ou de l'eugénol et/ou de l'eucalyptol et/ou des extraits de myrrhe et/ou des extraits de sauge et/ou des extraits de clou de girofle et/ou des extraits de thé et/ou des extraits de propolis et/ou des extraits d'échinacée.

8. Préparation selon l'une des revendications 1 à 7 pour l'utilisation dans la prévention et le traitement de caries et/ou dans la lutte contre l'halitose et/ou le traitement de la gingivite ou la parodontite, la préparation se présentant sous la forme d'une solution de bain de bouche, la solution de bain de bouche étant introduite dans la cavité buccale où elle est laissée pendant une durée d'au moins 10 secondes, de préférence d'au moins 20 secondes et notamment d'au moins 45 secondes.
